# EUROPEAN PATENT SPECIFICATION

(11) **EP 2 424 489 B1**
(45) Date of publication and mention of the grant of the patent: **05.10.2016**
(21) Application number: 10718876.5
(22) Date of filing: 23.04.2010
(51) Int. Cl.: A61Q 5/12, A61K 8/64

(54) **CONDITIONING COMPOSITION FOR HAIR**
PFLEGEZUSAMMENSETZUNG FÜR DIE HAARE
COMPOSITION DE TRAITEMENT DES CHEVEUX

(30) Priority: 27.04.2009 EP 09005820
(43) Date of publication of application: 07.03.2012
(73) Proprietor: Kao Germany GmbH, 64297 Darmstadt (DE)
(72) Inventor: HOFFMANN, Martin, 64673 Zwingenberg (DE); GRIT, Mustafa, 64579 Gernsheim (DE)
(86) International application number: PCT/EP2010/002509
(87) International publication number: WO 2010/124817

(56) References cited:
- EP-A- 1 297 830
- WO-A-2007/068401
- US-A1- 2008 095 732

## Description

The present invention is related to a conditioning composition for hair comprising at least one hair conditioning agent and carnosine.

Conditioning compositions for hair have been known for ages. Various types of conditioners are available on the market and new ones are being introduced almost every day. Although the extremely developed conditioner market, there is still need for improvements.

It is known that consumers with damaged hair are often not satisfied with hair conditioning effect of known conditioners. In case they choose a rich conditioner, the hair afterwards looses its volume and/or body and after a lighter conditioner, hair is not conditioned enough so that it is not combable, does not appear shiny and it is not manageable, etc. In other words, for consumers with damaged hair which is not homogeneous in degree of damage, it is very hard to find a correct conditioner.

On the other hand, fine hair has to be correctly conditioned for improving combability and shine, but this often results in loss of volume and partly body because of, especially damaged fine hair, heavy conditioning ingredients load onto hair. This is certainly not satisfying for consumers.

Therefore, there is a great need for improved conditioner compositions which homogeneously conditions damaged and healthy hair, especially damaged fine hair and does not result in loss of volume and body without changing combing shine and other properties of hair negatively.

WO 2007/068401 A1 discloses hair conditioning compositions comprising an oligopeptide, most preferably glycylglycine, and cationic amidoamine conditioning surfactants such as stearamidopropyldimethylamine.

It has surprisingly been found out that a composition comprising at least one hair conditioning agent and carnosine conditions damaged and healthy hair excellently homogeneously so that hair becomes combable, has excellent grip, shine, elasticity, volume, body and manageable.

WO 2007/068401 A1 discloses hair treatment compositions comprising a combination of a sugar, an oligopeptide i.e. glycylglycine, and an aminoacid. However the document does not disclose a composition comprising cationic or cationizable surfactant and carnosine.

Accordingly, the first object of the present invention is a conditioning composition for hair comprising at least one hair conditioning agent selected from compounds according to general structure

R₁-A-R₂-B

wherein R₁ is a saturated or unsaturated, straight or branched alkyl group with 8 to 24 C atoms, R₂ is a straight or branched alkyl group with 1 to 4 C atoms, A is a group selected from O, 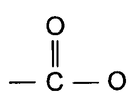 and 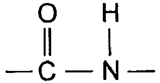 , preferably an O and B is selected from 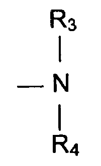 wherein R₃ and R₄ are the same or different is H or an alkyl with 1 to 4 C atoms, hydroxyl alkyl with 1 to 4 C atoms and di hydroxyl alkyl with 2 to 4 C atoms, 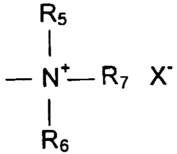 R₅, and R₆ are the same or different, an alkyl with 1 to 4 C atoms, hydroxyl alkyl with 1 to 4 C atoms and di hydroxyl alkyl with 2 to 4 C atoms, R₇ is an alkyl with 1 to 4 C atoms, hydroxyl alkyl with 1 to 4 C atoms or di hydroxyl alkyl with 2 to 4 C atoms and

-R₂-A-R₁

wherein R₁, A and R₂ have the above meaning and X is chloride, bromide, methosulfate, and carnosine.

Further object of the present invention is the use of the composition of the present invention for conditioning hair and especially to keep or improve combability, shine, volume, body, elasticity and manageability of hair.

Still further object of the present invention is the method of conditioning hair wherein hair is treated with a composition of the present invention and optionally rinsed off from hair after a processing time of up to 30 min.

Compositions of the present invention are suitable for either rinse off or leave in applications. Further object of the present invention is process for conditioning hair wherein a composition according to present invention is applied onto hair and not rinsed off.

Carnosine is a dipeptide of β-alamine and L-histidine.

Concentration of carnosine is in the range of 0.01 to 5%, preferably 0.05 to 3% and more preferably 0.1 to 2.5% and most preferably 0.2 to 1.5% by weight calculated to the total composition.

Composition of the present invention comprises at least one hair conditioning agent. Hair conditioning agents are selected from cationic and/or cationizable surfactants according to above general structure. In the preferred embodiment of the present invention, R₁ is saturated or unsaturated, straight or branched alkyl group with 10 to 24C atoms, more preferably 12 to 22 C atoms and R₂ is straight or branched alkyl group with 1 to 4 C atoms, A, B, R₃ to R₇ are same as above.

Non-limiting suitable examples are stearyloxypropyl amine, palmityloxypropyl amine, stearyloxypropyldimethyl amine, stearyloxypropyldiethyl amine, stearyloxyethylyldimethyl amine, stearyloxyethyl amine, myristyloxypropyl amine, myristyloxypropyldimethyl amine, palmitamidopropyl amine, palmitamidopropyl methylamine, palmitamidopropyl diethylamine, palmitamidopropyl dibutylamine, palmitamidopropyl buylamine, palmitamidopropyl dipropylamine, palmitamidopropyl propylamine, palmitamidopropyl dihydroxyethylamine, palmitamidopropyl hydroxyethylamine, palmitamidopropyl dihydroxypropylamine, palmitamidopropyl hydroxypropylamine, lauramidopropyl amine, lauramidopropyl methylamine, lauramidopropyl diethylamine, lauramidopropyl dibutylamine, lauramidopropyl buylamine, lauramidopropyl dipropylamine, lauramidopropyl propylamine, lauramidopropyl dihydroxyethylamine, lauramidopropyl hydroxyethylamine, lauramidopropyl dihydroxypropylamine, lauramidopropyl hydroxypropylamine, stearamidopropyl amine, stearamidopropyl methylamine, stearamidopropyl diethylamine, stearamidopropyl dibutylamine, stearamidopropyl butylamine, stearamidopropyl dipropylamine, behenamidopropyl propylamine, behenamidopropyl dihydroxyethylamine, behenamidopropyl hydroxyethylamine, behenamidopropyl dihydroxypropylamine, behenamidopropyl hydroxypropylamine, behenamidopropyl amine, behenamidopropyl methylamine, behenamidopropyl diethylamine, behenamidopropyl dibutylamine, behenamidopropyl butylamine, behenamidopropyl dipropylamine, behenamidopropyl propylamine, behenamidopropyl dihydroxyethylamine, behenamidopropyl hydroxyethylamine, behenamidopropyl dihydroxypropylamine, behenamidopropyl hydroxypropylamine, dipalmitamidopropyl methylamine, dipalmitamidopropyl ethylamine, dipalmitamidopropyl butylamine, dipalmitamidopropyl propylamine, dipalmitamidopropyl hydroxyethylamine, dipalmitamidopropyl hydroxypropylamine, dilauramidopropyl amine, dilauramidopropyl methylamine, dilauramidopropyl buylamine, dilauramidopropyl hydroxyethylamine, dilauramidopropyl hydroxypropylamine, distearamidopropyl amine, distearamidopropyl methylamine, dibehenamidopropyl propylamine, dibehenamidopropyl hydroxyethylamine, palmitoamidopropyl trimethyl ammonium chloride, stearamidopropyl trimethylammonium chloride, behenamidopropyl tri hydroxyethalmonium chloride, distearylamidopropyl dimethyl ammonium chloride, dicetylamidodihydroxyethyl ammonium chloride, palmitoylpropyl amine, palmitoylpropyl methylamine, palmitoylpropyl diethylamine, palmitoylpropyl dibutylamine, palmitoylpropyl buylamine, palmitoylpropyl dipropylamine, palmitoylpropyl propylamine, palmitoylpropyl dihydroxyethylamine, palmitoylpropyl hydroxyethylamine, palmitoylpropyl dihydroxypropylamine, palmitoylpropyl hydroxypropylamine, myristoylpropyl amine, myristoylpropyl methylamine, myristoylpropyl diethylamine, myristoylpropyl dibutylamine, myristoylpropyl buylamine, myristoylpropyl dipropylamine, myristoylpropyl propylamine, myristoylpropyl dihydroxyethylamine, myristoylpropyl hydroxyethylamine, myristoylpropyl dihydroxypropylamine, myristoylpropyl hydroxypropylamine, stearoylpropyl amine, stearoylpropyl methylamine, stearoylpropyl diethylamine, stearoylpropyl dibutylamine, stearoylpropyl butylamine, stearoylpropyl dipropylamine, behenylpropyl propylamine, behenylpropyl dihydroxyethylamine, behenylpropyl hydroxyethylamine, behenylpropyl dihydroxypropylamine, behenylpropyl hydroxypropylamine, behenylpropyl amine, behenylpropyl methylamine, behenylpropyl diethylamine, behenylpropyl dibutylamine, behenylpropyl butylamine, behenylpropyl dipropylamine, behenylpropyl propylamine, behenylpropyl dihydroxyethylamine, behenylpropyl hydroxyethylamine, behenylpropyl dihydroxypropylamine, behenylpropyl hydroxypropylamine, dipalmitoylpropyl methylamine, dipalmitoylpropyl ethylamine, dipalmitylpropyl butylamine, dipalmitylpropyl propylamine, dipalmitylpropyl hydroxyethylamine, dipalmitylpropyl hydroxypropylamine, dilauroylpropyl amine, dilauroylpropyl methylamine, dilauroylpropyl buylamine, dilauroylpropyl hydroxyethylamine, dilauroylpropyl hydroxypropylamine, distearylpropyl amine, distearylpropyl methylamine, dibehenylpropyl propylamine, dibehenylpropyl hydroxyethylamine, palmitylpropyl trimethyl ammonium chloride, stearylpropyl trimethylammonium chloride, behenylpropyl tri hydroxyethalmonium chloride, distearylpropyl dimethyl ammonium chloride, dicetyldihydroxyethyl ammonium chloride, dioleoylethylhydroxyethylmonium methosulfate and dicocoylethylhydroxyethylmonium methosulfate.

Preferred are the compounds stearyloxypropyl amine, palmityloxypropyl amine, stearyloxypropyldimethyl amine, stearyloxypropyldiethyl amine, stearyloxyethylyldimethyl amine, stearyloxyethyl amine, myristyloxypropyl amine, myristyloxypropyldimethyl amine, stearyloxypropyl trimethyl ammonium chloride, palmityloxypropyl trimethyl ammonium chloride, stearyloxypropyl trimethyl ammonium chloride, stearyloxypropyltriethyl ammonium chloride, stearyloxyethylyltrimethyl ammonium chloride, stearyloxyethyl trimethyl ammonium chloride, myristyloxypropyl trimethyl ammonium chloride and myristyloxypropyltrimethyl ammonium chloride. More preferred are the ones with palmityl and stearyl alkyl groups and most preferred are the ones with stearyl group.

Concentration of the above compounds in the compositions of the present invention is in the range of 0.01 to 20%, preferably 0.02 to 15%, more preferably 0.05 to 10% and most preferably 0.1 to 7.5% and in particular 0.25 to 5% by weight calculated to total composition.

Composition of the present invention comprises preferably additional conditioning agents. Additional conditioning agents are selected from cationic quaternary ammonium surfactants, cationic polymers, oil and/or oily substances such as silicones, natural and synthetic oils, fatty alcohol ethers, fatty acid fatty alcohol esters and polyols such as panthenol, glycerine, polyethyleneglycols, polypropyleneglycols.

Cationic quaternary ammonium surfactants are preferably according to general structure 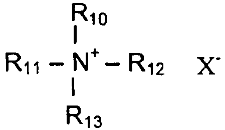 where R₁₀ is a saturated or unsaturated, branched or non-branched alkyl chain with 8-24 C atoms, preferably 10 to 24 C atoms and more preferably 12 to 22 C atoms, and R₁₁ is unsaturated or saturated, branched or non-branched alkyl chain with 1 - 24 C atoms and R₁₂ and R₁₃ are lower alkyl chain with 1 to 4 carbon atoms which may be substituted with one or more hydroxyl groups, and X is anion such as chloride, bromide, methosulfate.

Nonlimiting suitable examples are cetyltrimethyl ammonium chloride, steartrimonium chloride, behentrimonium chloride, myristyltrimethyl ammonium chloride, distearyldimethyl ammonium chloride, and dibehenyldimethyl ammonium chloride.

Concentration of at least one cationic quaternary ammonium surfactant compound according to the above general structure is in the range of 0.01 to 20%, preferably 0.02 to 15%, more preferably 0.05 to 10% and most preferably 0.1 to 7.5% and in particular 0.25 to 5% by weight calculated to total composition.

In one of the preferred from of the present invention, conditioning compositions comprise at least one cationic polymer as conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87 as well as silicone quaternium-1, silicone quaternium-2, silicone quaternium-2 panthenol succinate, silicone quaternium-3, silicone quaternium-4, silicone quaternium-5, silicone quaternium-6, silicone quaternium-7, silicone quaternium-8, silicone quaternium-9, silicone quaternium-10, silicone quaternium-11, silicone quaternium-12, silicone quaternium-15, silicone quaternium-16, silicone quaternium-16/Glycidoxy Dimethicone Crosspolymer, silicone quaternium-17, silicone quaternium-18, silicone quaternium-20 and silicone quaternium-21.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCl name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Concentration of at least one cationic polymer is in the range of 0.01 to 5%, preferably 0.01 to 3%more preferably 0.02 to 2.5% and most preferably 0.05 to 2% by weight calculated to total composition.

Oil and/or oily substances are selected from such as silicone oils, either volatile or non-volatile, natural and synthetic oils, fatty alcohol ethers (dialkyl ethers) and fatty acid fatty alcohol esters. Among silicone oils those can be added to the compositions include dimethicone, dimethiconol, polydimethylsiloxane, DC fluid ranges from Dow Corning, arylated silicones such as phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxane, aminated silicones such as amodimethicone, aqueous emulsion of divinyldimethicone/dimethicone copolymer, preferably with a viscosity of higher than 1 x 10⁸ mm²/s, more preferably higher than 1.1 x 10⁸ mm²/s, and most preferably higher than 1.2 x 10⁸ mm²/s measured at 0.01 Hz and at approximately 25°C.

Concentration of one or more silicone oils is in the range of 0.01 to 5%, preferably 0.01 to 3%more preferably 0.02 to 2.5% and most preferably 0.05 to 2% by weight calculated to total composition.

Natural and/or synthetic oils are comprised in the compositions of the present invention at a concentration in the range of 0.01 to 5%, preferably 0.01 to 3%more preferably 0.02 to 2.5% and most preferably 0.05 to 2% by weight calculated to total composition. Suitable non-limiting examples are such as olive oil, almond oil, avocado oil, wheatgerm oil, ricinus oil and synthetic oils, such as mineral oil.

Fatty acid fatty alcohol esters as oily substances are comprised in the compositions of the present invention at a concentration in the range of 0.01 to 5%, preferably 0.01 to 3%more preferably 0.02 to 2.5% and most preferably 0.05 to 2% by weight calculated to total composition. Suitable non-limiting examples are such as isopropyl myristate, palmitate, stearate and isostearate, oleyl oleate, isocetyl stearate, hexyl laurate, dibutyl adipate, dioctyl adipate, myristyl myristate and oleyl erucate.

Fatty alchol ethers (dialkyl ethers) are comprised in the compositions of the present invention at a concentration in the range of 0.01 to 5%, preferably 0.01 to 3%more preferably 0.02 to 2.5% and most preferably 0.05 to 2% by weight calculated to total composition. Suitable non-limiting examples are such as dimyristyl ether, dicetyl ether and dicaprylyl ether.

In the preferred from of the present invention, compositions comprise at least one silicone oil, at least one synthetic or natural oil, at least one fatty alcohol ether or fatty acid fatty alcohol ester at a total concentration in the range of 0.01 to 10%, preferably 0.05 to 7.5%, more preferably 0.1 to 5 and most preferably 0.1 to 3% by weight calculated to total composition. The concentration ranges mentioned above for the individual ones are also valid in this paragraph mentioned preferred embodiment of the present invention in addition to the total concentration mentioned in this paragraph.

Conditioning agent is also at least one polyol such as panthenol, glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₈ CO (O CH₂ CH₂)ₙ OH

or

R₈ CO (O CH₂ CH₂)ₙ O OC R₉

where R₈ and R₉ are independent from each other saturated, unsaturated or branched or non-branched alkyl chain with 7 to 21 C atoms and n is typically 2 - 100.

In especially preferred embodiment of the present invention, conditioning composition comprises in addition to the dipeptide, at least once cationic and/or cationizable surfactant compound, at least one cationic polymer, at least one silicone oil, at least one at least one synthetic or natural oil, at least one fatty alcohol ether or fatty acid fatty alcohol ester and preferably at a total concentration in the range of 0.01 to 10%, preferably 0.05 to 7.5%, more preferably 0.1 to 5 and most preferably 0.1 to 3% by weight calculated to total composition.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Composition of the present invention may further comprise at least one polyphenol or mixture of polyhenols. Polyphenols are included into compositions of the present invention from a natural plant extract. In principal any natural plant extract rich of polyphenols is suitable within the meaning of the present invention. Within the meaning of the present invention the extracts are liquid extracts and prepared by mixing plant parts such as leaves, fruits, blossoms and roots with a solvent such as water, alcohol, propyleneglycol or mixture of more than one solvent and incubating for certain period of time and filtrating the undissolved plant parts. Suitable aqueous (e.g. steam-distilled) alcoholic or hydro-alcoholic plant extracts known per se are in particular aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis", 4th Ed. Preferred plant extracts are prepared from Vitis vinifera, Malus domestica, Camelia sinensis, Juglans regia Ribes Uva-Crispa, Ribes nigrum, Ribes rubrum and Punica granatum. The above mentioned extracts may also be available in the powder form and such are also suitable within the meaning of the present invention.

The polyphenol comprising extracts are included into the compositions of the present invention at a concentration of 0.001 to 10%, preferably 0.005 to 7.5%, more preferably 0.01 to 5% and most preferably 0.05 to 2.5% by weight, calculated to total composition based on dry matter of the extract.

Further in preferred embodiment of the present invention, compositions comprise at least one UV filter and at least one ubichinone of the following formula 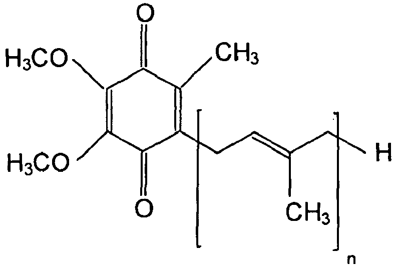 where n is a number between 1 and 10. It should be noted that the compositions of the present invention can certainly comprise more than one ubichinone. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Compositions of the present invention preferably comprise at least one UV filter. Principally any substance known as UV filter is suitable for the compositions of the present invention. Non-limiting examples are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15. Above mentioned UV filters are those oil and water soluble ones for the purpose of protecting hair colour. In other words, anionic and nonionic, oily, UV filters are suitably used in the compositions of the present invention. In the preferred from of the invention the compositions comprise at least one water soluble UV filter and at least one oil soluble one. Further preferred that both UV filters are present at a weight ratio in the range of oil soluble to water soluble UV filter 1:10 to 10:1, preferably 1:5 to 5:1, more preferably 1:3 to 3:1 and most preferably 1:1 in the compositions of the present invention.

The amount of the UV-absorber as a total ranges typically from about 0.01 % to 5%, preferably 0.05 to 3%, more preferably from 0.05 % to 2.5% and most preferably from 0.1 % to 2 % by weight, calculated to the total composition.

In another preferred form of the invention, conditioning composition can comprise one or more organic solvent such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, poypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are ethanol, isopropanol, benzylalcohol and polypropylene glycols. Concentration of organic solvents should not exceed 50% by weight, preferably in the range of 0.1 to 40 %, more preferably 0.1 to 30% by weight and most preferably 0.1 to 20% by weight calculated to total composition. It should be noted that concentration of at least one organic solvent is very much dependent on the type of preparation i.e. a solution can contain higher concentration of organic solvent than an emulsion composition.

Conditioning composition of the present invention comprises at least one glyceryl ether of the following formula 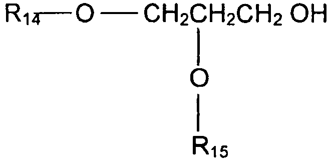 wherein R₁₄ is straight or branched, saturated or unsaturated alkyl chain with 4 to 24 C atoms, preferably 4 to 18 and more preferably 4 to 12 C atoms and R₁₅ is H, or straight or branched, saturated or unsaturated alkyl chain with 4 to 24 C atoms, preferably 4 to 18 and more preferably 4 to 12 C atoms and most preferably R₅ is H, at a concentration of 0.1 to 10%, preferably 0.1 to 5% and more preferably 0.25 to 3% and most preferably 0.5 to 2.5% by weight calculated to total composition.

Suitable unlimited examples are glyceryl butyl ether, glyceryl isobutyl ether, glyceryl tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether, glyceryl lauryl ether, glyceryl myristyl ether, glyceryl palmityl ether, glyceryl stearyl ether and glyceryl behenyl ether and their mixtures. Most preferred are glyceryl butyl ether, glyceryl isobutyl ether, glyceryl tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether are glyceryl lauryl ether, and their mixtures.

It should be noted that within the disclosure of the present description, gylceryl decyl ether is used as synonym of decyl glycerine. For the other compounds in the above paragraph the same is valid.

Further in preferred embodiment of the present invention, compositions comprise at least one direct dye. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuffs is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the preferred anionic dyestuffs are Acid Red 52, Acid Violet 2, Acid Red 33, Acid Orange 4, Acid Red 27 and Acid Yellow 10 and their salts. The most preferred anionic dyes are Acid Red 52, Acid Violet 2, Acid Red 33, Acid Orange 4 and Acid Yellow 10, and their salts

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No. 15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of one or more direct dyes in total is in the range of 0.001 to 5% by weight, preferably 0.01 to 4% more preferably 0.05 to 3% and most preferably 0.1 to 2.5% by weight calculated to total composition.

Conditioning compositions of the present invention can comprise at least one surfactant selected from anionic, non-ionic and amphoteric surfactants, preferably at a concentration range of 0.5 to 20%, preferably 1 to 15% and more preferably 1 to 10% by weight, calculated to the total composition. At least one surfactant is added into the composition of the present invention as solubilizer and/or emulsifier. The most preferred surfactant is nonionic surfactants.

Among suitable examples, suited are alkyl polyglucosides of the general formula

R₁₇-O-(R₁₈O)ₙ-Zₓ,

wherein R₁₇ is an alkyl group with 8 to 18 carbon atoms, R₁₈ is an ethylene or propylene group, Z is a saccharide group with 5 to 6 carbon atoms, n is a number from 0 to 10 and x is a number between 1 and 5.

Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further nonionic surfactants useful in the compositions of the present invention are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

Conditioning compositions of the present invention can be in the form of emulsions, solutions, gels and dispersions. In the case that solutions and/or gels forms are preferred the appearance can be either with a transparent or opaque. As a product form, foam is as well suited when packed into a pressurized can or delivered through a pump-foamer (non-aerosol). In the case that an aerosol foam preparation is preferred, propellant gas must be added to the formulation. The suitable propellant gasses are carbondioxide, dimethylether and alkanes such as butane, propane, isobutane or their mixtures.

The emulsion type of conditioning compositions comprise preferably additionally at least one fatty alcohol of the following formula

R₂₀-OH

where R₂₀ is a saturated or unsaturated, branched or non-branched fatty acyl chain with 8 - 24 C atoms. Concentration of fatty alcohols is usually less than 20%, preferably less than 15% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

Conditioning compositions of the present invention can comprise additionally any compound customarily found in conditioning compositions such as chelating agents, preservatives and fragrance.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

The pH of the compositions according to the present invention is suitably between 2 and 8 and preferably in the range of 2.5 to 6.5, more preferably 3 to 5.5 and most preferably 3.5 to 5.

In principal pH of the compositions can be adjusted with any organic and/or inorganic acids or their mixture. Some of them to mention are phosphoric acid, hydrochloric acid as the inorganic ones and to the organic acids the well known citric acid and lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. It has further been observed that improved conditioning and brightening performance was observed when compositions comprise at the same time at least one hydroxycarboxylic and/or dicarboxylic acids.

Viscosity of the conditioning composition may be adjusted according to the application form and generally should not be more than 100,000 mPa.s at 20°C measured with Brookfield Rheometer at a shear rate of 10 sec⁻¹.

Thickening agents may be added into the compositions of the present invention. Suitable non-limiting examples are cellulose derivatives such as hydroxylcellulose, methyl cellulose, hydroxymethlyl cellulose, hydroxypropyl cellulose, xanthan gum, guar gum and its derivatives especially nonionic ones as thickener, which should not principally exclude to use cationic ones as thickener and conditioner, such hydroxypropyl guar, acrylates as the synthetic ones which may be alone or in combination with others available from various suppliers known as Carbomer with its INCl name and their derivatives. Thickeners may be included at a concentration of 0.05 to 2.5% by weight calculated to total composition. Concentration of thickener is very much dependent on the thickener its self and also the preparation such as pH value of the composition etc.

In a preferred embodiment of the present invention the compositions are present in a kit form for conditioning hair. Accordingly present invention is a kit for hair comprising two or more products wherein at least one product comprises a composition according to the present invention.

The following examples are to illustrate the invention, but not to limit. The compositions according to the invention are prepared by mixing the individual components, whereby it is also possible to use pre-mixtures of various ingredients.

### Example 1

| | % by weight |
|---|---|
| Stearoxypropyl trimethylammonium methosulfate | 1.5 |
| Carnosine | 0.7 |
| Citric acid | q.s. pH 4.0 |
| Fragrance, preservative | q.s. |
| Water | to 100 |

The above composition was prepared by dissolving carnosine in water and subsequently dispersing cationic surfactant in the solution and adjusting the pH of the composition using citric acid.

Above composition was used as a leave in spray composition and applied onto towel dried hair after shampooing and not rinsed off after application. The composition improves combability, shine, elasticity, volume and body of damaged hair. In addition it was observed that the same properties of hair which included highly damaged tips and root area was relatively healthy, were improved as well.

### Example 2

| | % by weight |
|---|---|
| Stearoxypropyl trimethylammonium methosulfate | 1 |
| Carnosine | 0.5 |
| Cetearyl alcohol | 7 |
| Ceteareth-20 | 3 |
| Citric acid | q.s. pH 4.0 |
| Fragrance, preservative | q.s. |
| Water | to 100 |

The above composition was prepared by emulsifying Cetearyl alcohol and ceteareth-20 in water at approximately 70°C and after cooling down to approximately 40°C the other ingredients were added.

The composition improves combability, shine, volume and body of damaged hair.

### Example 3

| | % by weight |
|---|---|
| Stearoxypropyl trimethylammonium methosulfate | 0.5 |
| Carnosine | 0.5 |
| Cetearyl alcohol | 7 |
| Cetrimonium chloride | 1.5 |
| Citric acid | q.s. pH 4.0 |
| Fragrance, preservative | q.s. |
| Water | to 100 |

The above composition was prepared in the same way as of Example 1.

The composition conditions inhomogeneously damaged hair homogeneously so that hair is combable, has shine, elasticity, volume and it is manageable.

### Example 4

| | % by weight |
|---|---|
| Stearoxypropyl trimethylammonium methosulfate | 0.5 |
| Carnosine | 0.5 |
| Behenyl alcohol | 5 |
| Ceteareth-20 | 2 |
| Polyquaternium-10 | 1 |
| Dimethicone | 0.5 |
| Lactic acid | q.s. pH 4.2 |
| Fragrance, preservative | q.s. |
| Water | to 100 |

### Example 5

| | % by weight |
|---|---|
| Stearoxypropyl trimethylammonium methosulfate | 0.5 |
| Carnosine | 0.5 |
| Polyquaternium-6 | 1 |
| Amodimethicone | 0.5 |
| Panthenol | 0.5 |
| Isopropylmyristate | 0.2 |
| Cetearyl alcohol | 5.0 |
| Ceteareth-20 | 1.5 |
| Lactic acid | q.s. pH 5.0 |
| Fragrance, preservative | q.s. |
| Water | to 100 |

The above composition was used as a rinse off conditioner after cleansing hair. In dry state it was observed that hair was easily combable, had elasticity, volume and body.

### Example 6

| | % by weight |
|---|---|
| Stearyloxypropyl amine | 0.5 |
| Carnosine | 0.5 |
| Polyquaternium-6 | 1 |
| Lactic acid | q.s. pH 5.0 |
| Fragrance, preservative | q.s. |
| Water | to 100 |

The above composition was used as a leave-in conditioner from a pump spray bottle on damaged hair which was freshly washed and towel dried. In dry state it was observed that hair was easily combable, had elasticity, volume and body.

### Example 7

| | % by weight |
|---|---|
| Stearyloxypropyl amine | 1 |
| Carnosine | 1 |
| Cetearyl alcohol | 7 |
| Ceteareth-20 | 3 |
| Dicetly ether | 0.2 |
| Benzophenone-3 | 0.4 |
| Panthenol | 0.5 |
| Phenyl trimethicone | 0.3 |
| Citric acid | q.s. pH 4.0 |
| Fragrance, preservative | q.s. |
| Water | to 100 |

The above composition was especially suitable for coloured and/or highlighted hair. The coloured hair treated with the above conditioner is exceptionally shiny, well combable and has elasticity, volume and body.

### Example 8

| | % by weight |
|---|---|
| Stearyloxypropyldimethyl amine | 0.5 |
| Carnosine | 0.2 |
| Cetearyl alcohol | 7 |
| Cetrimonium chloride | 1.2 |
| Benzophenone-3 | 0.4 |
| Polyquaternium-10 | 0.5 |
| Panthenol | 0.5 |
| Phenyl trimethicone | 0.2 |
| Citric acid | q.s. pH 4.0 |
| Fragrance, preservative | q.s. |
| Water | to 100 |

The above composition was especially suitable for coloured and/or highlighted hair. The coloured hair treated with the above conditioner is exceptionally shiny, well combable and has elasticity, volume and body.

### Example 9

| | % by weight |
|---|---|
| Stearyloxypropyldimethyl amine | 1 |
| Carnosine | 0.7 |
| DC HMW 2220 | 0.2 |
| Cetearyl alcohol | 7 |
| Ceteareth-20 | 3 |
| Benzophenone-4 | 0.2 |
| Isopropyl myristate | 0.2 |
| Glycerin | 3 |
| Guar hydroxyproly trimonium chloride | 0.3 |
| Basic red 51 | 0.1 |
| Citric acid | q.s. pH 4.2 |
| Fragrance, preservative | q.s. |
| Water | to 100 |

Above conditioner gives dark blonde hair red shimmer.

### Example 10

| | % by weight |
|---|---|
| Stearyloxypropyldimethyl amine | 1 |
| Carnosine | 0.5 |
| Cetearyl alcohol | 7 |
| Ceteareth-20 | 3 |
| Octylmethoxy cinnamate | 0.4 |
| Glycerin | 3 |
| Trimethyl pentaphenyl trisiloxane | 0.3 |
| Basic red 51 | 0.01 |
| Basic yellow 87 | 0.1 |
| Basic orange 31 | 0.02 |
| Citric acid | q.s. pH 4.2 |
| Fragrance, preservative | q.s. |
| Water | to 100 |

Above conditioner gives light blonde hair additional blond shine.

### Example 11

| | % by weight |
|---|---|
| Stearyloxypropyldimethyl amine | 1 |
| Carnosine | 0.4 |
| Cetearyl alcohol | 7 |
| Ceteareth-20 | 3 |
| Octylmethoxy cinnamate | 0.4 |
| Glycerin | 3 |
| Phenyl trimethicone | 0.3 |
| Coenzyme Q10 | 0.08 |
| Vitis vinifera (dry matter) | 0.1 |
| Ethyl hexyl glycerin | 0.5 |
| Citric acid | q.s. pH 4.2 |
| Fragrance, preservative | q.s. |
| Water | to 100 |

Above conditioner enhances combability, shine, volume body and elasticity. Hair treated with the above conditioner is excellently manageable and easily styled.

### Example 12

| | % by weight |
|---|---|
| Stearyloxypropyldimethyl amine | 1 |
| Polyquaternium-6 | 0.2 |
| Cetearyl alcohol | 7 |
| Steartrimonium chloride | 1 |
| Glycerin | 3 |
| Carnosine | 0.6 |
| Dimethicone | 0.3 |
| Coenzyme Q10 | 0.08 |
| Isopropyl myristate | 0.2 |
| Lactic acid | q.s. pH 3.8 |
| Fragrance, preservative | q.s. |
| Water | to 100 |

Above conditioner enhances combability, shine, volume body and elasticity. Hair treated with the above conditioner is excellently manageable and easily styled. Above conditioner was also used as leave in conditioner and bodyfying effects was especially enhanced.

### Example 13

| | % by weight |
|---|---|
| Stearyloxypropyldimethyl amine | 1 |
| Carnosine | 0.2 |
| Cocamidoproyl betaine | 0.8 |
| Steartrimonium chloride | 1 |
| Glycerin | 3 |
| Coenzyme Q10 | 0.08 |
| Isopropyl myristate | 0.1 |
| Lactic acid | q.s. pH 3.8 |
| Fragrance, preservative | q.s. |
| Water | to 100 |

Above composition had a viscosity below 500 mPa.s and was filled into a pump foamer and used as leave in conditioner on a freshly washed and towel dried hair. Hair was excellently shiny and bodified also excellently combable.

The above composition was also used as aerosol foam and therefore filled inot an aerosol can with 10% propane-butane mixture as a propellant. The above results were confirmed.

The above composition can also be used as a pump spray.

### Example 14

| | % by weight |
|---|---|
| Cetylstearylalcohol | 5.0 |
| Stearyloxypropyltrimethyl ammonium chloride | 1.0 |
| Ceteareth 20 | 1.0 |
| Panthenol | 0,4 |
| Carnosine | 0.75 |
| Hydroxypropyl Guar Hydroxypropyltrimonium Chloride | 1.0 |
| Ethylhexyl glycerin | 0.8 |
| Tetramethyl tetraphenyl trisiloxane | 0.2 |
| Ribes nigrum (dry matter) | 0.1 |
| Avocado extract | 0.5 |
| Fragrance, preservative | q.s. |
| Malic acid | q.s. to pH 3.5 |
| Wasser | ad 100.0 |

### Example 15

| | % by weight |
|---|---|
| Cetylstearylalcohol | 5.0 |
| Stearyloxypropyltrimethylammoniumchlorid | 1.0 |
| Carnosine | 0.3 |
| Stearamidopropyldimethyl amine | 1.0 |
| Benzylalcohol | 2.5 |
| Trimethyl pentaphenyl trisiloxane | 0.3 |
| Ethylhexyl glycerin | 0.9 |
| Fragrance, preservative | q.s. |
| Lactic acid | q.s. pH 3.5 |
| Wasser | ad 100.0 |

Above composition is applied onto shampooed hair and processed for 5 min and rinsed off from hair. It was observed that wet hair is easily combable. In the dry state combability, manageability, elasticity and shine were very much improved.

Furthermore into the above conditioner composition, hair direct dye Basic red 51 was included. After use on dark blonde hair am excellent red shine was observed on the hair.

### Example 16

### Foam conditioner

| | % by weight |
|---|---|
| Stearyloxypropyldimethyl amine | 0.2 |
| Polyquaternium-11 | 0.7 |
| PEG-60-hydrogenated ricinus oil | 0.5 |
| Diphenylsiloxy phenyl trimethicone | 0.5 |
| Ethylhexyl glycerin | 1.2 |
| Carnosine | 0.7 |
| Stearamidopropyldimethyl amine | 1.0 |
| Malus domestica (dry matter) | 0.1 |
| Ubichinone | 0.075 |
| Benzophenone-3 | 0.3 |
| Fragrance, preservative | q.s. |
| Lactic acid | q.s. to pH 3.4 |
| Wasser | ad 100.0 |

pH of the composition is adjusted to 3.4. The composition is suitable for leave-in and rinse off. In leave-in application, amount used is obviously less than in the case of a rinse of application. The composition is packed into an aerosol can with 90/10 ratio, by weight, liquid composition to propellant. As propellant propane, butane mixture is used.

Into the above composition 0.1 % Acid red 52 was added. It was possible to realize red shimmer onto dark blonde hair.

## Claims

1. Conditioning composition for hair **characterized in that**, it comprises at least one hair conditioning agent selected from compounds according to general structure
R₁-A-R₂-B
wherein R₁ is a saturated or unsaturated, straight or branched alkyl group with 8 to 24 C atoms, R₂ is a straight or branched alkyl group with 1 to 4 C atoms, A is a group selected from O, 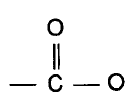 and 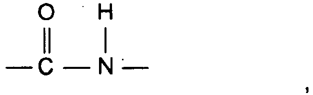 preferably O, and B is selected from 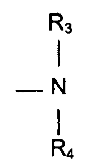 wherein R₃ and R₄ are the same or different is H or an alkyl with 1 to 4 C atoms, hydroxyl alkyl with 1 to 4 C atoms and di hydroxyl alkyl with 2 to 4 C atoms, 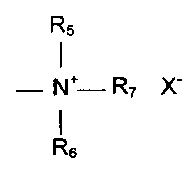 R₅, and R₆ are the same or different, an alkyl with 1 to 4 C atoms, hydroxyl alkyl with 1 to 4 C atoms and di hydroxyl alkyl with 2 to 4 C atoms, R₇ is an alkyl with 1 to 4 C atoms, hydroxyl alkyl with 1 to 4 C atoms or di hydroxyl alkyl with 2 to 4 C atoms and
-R₂-A-R₁
wherein R₁, A and R₂ have the above meaning and X is chloride, bromide, methosulfate, and carnosine.

2. Composition according to claim 1 **characterised in that** it comprises at least one hair conditioning agent selected from compounds according to general structure at a concentration in the range of 0.01 to 20% by weight, calculated to total composition.

3. Composition according to claims 1 and 2 **characterized in that** it comprises carnosine at a concentration of 0.01 to 5% by weight calculated to total composition.

4. Composition according to any of the preceding claims **characterized in that** it comprises additional hair conditioning agent selected from cationic surfactants according to general structure 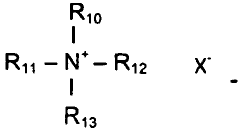 where R₁₀ is a saturated or unsaturated, branched or non-branched alkyl chain with 8-24 C atoms, preferably 10 to 24 C atoms and more preferably 12 to 22 C atoms, and R₁₁ is unsaturated or saturated, branched or non-branched alkyl chain with 1 - 24 C atoms and R₁₂ and R₁₃ are lower alkyl chain with 1 to 4 carbon atoms which may be substituted with one or more hydroxyl groups, and X is anion such as chloride, bromide, methosulfate, cationic polymers, oil and/or oily substances and polyols.

5. Composition according to claims 4 **characterized in that** oil and/or oily substances selected from silicone oils, fatty alcohol ethers (dialkyl ether), fatty acid fatty alcohol esters.

6. Composition according to any of the preceding claims **characterised in that** it comprises at least one direct dye.

7. Composition according to any of the preceding claims **characterised in that** it comprises at least one fatty alcohol of the following formula
R₂₀-OH
where R₂₀ is a saturated or unsaturated, branched or non-branched fatty acyl chain with 8 - 24 C atoms and optionally at least one nonionic surfactant as an emulsifier.

8. Composition according any of the preceding claims **characterized in that** it comprise one or more of compounds selected from protein hyrolyzate, polyphenol, ubichinone of the general structure 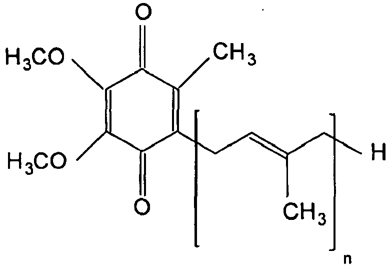 where n is a number between 1 and 10 , UV filter, organic solvent, thickening agent and chelating agent.

9. Composition according to any of the preceding claims **characterized in that** it has a pH in the range of 2.0 to 8.0.

10. Use of the composition according to claims 1 to 9 to condition hair and especially to keep or improve shine, volume, body, elasticity and manageability of hair.

11. Kit for hair **characterised in that** it comprises two or more products wherein at least one product comprises a composition according to claims 1 to 9.

## Patentansprüche

1. Konditionierzusammensetzung für Haar, **dadurch gekennzeichnet, dass** sie mindestens ein Haarkonditionierungsmittel aufweist, ausgewählt aus Verbindungen gemäß der allgemeinen Struktur
R₁-A-R₂-B
wobei R₁ für eine gesättigte oder ungesättigte, lineare oder verzweigte Alkylgruppe mit 8 bis 24 C-Atomen steht, R₂ für eine lineare oder verzweigte Alkylgruppe mit 1 bis 4 C-Atomen steht, A für eine Gruppe steht, die ausgewählt ist aus O, 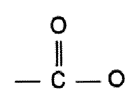 und 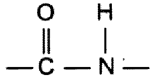 vorzugsweise O, und B ausgewählt ist aus 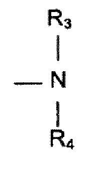 wobei R₃ und R₄ gleich oder verschieden sind und für H oder ein Alkyl mit 1 bis 4 C-Atomen, ein Hydroxyalkyl mit 1 bis 4 C-Atomen und ein Dihydroxyalkyl mit 2 bis 4 C-Atomen stehen, 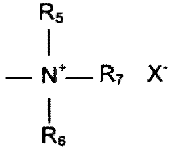 wobei R₅ und R₆ gleich oder verschieden sind und für ein Alkyl mit 1 bis 4 C-Atomen, ein Hydroxyalkyl mit 1 bis 4 C-Atomen und ein Dihydroxyalkyl mit 2 bis 4 C-Atomen stehen, R₇ für ein Alkyl mit 1 bis 4 C-Atomen, ein Hydroxyalkyl mit 1 bis 4 C-Atomen oder ein Dihydroxyalkyl mit 2 bis 4 C-Atomen steht, und
-R₂-A-R₁
wobei R₁, A und R₂ die obige Bedeutung haben und X für Chlorid, Bromid, Methosulfat und Carnosin steht.

2. Zusammensetzung nach Anspruch 1, **dadurch gekennzeichnet, dass** sie mindestens ein Haarkonditionierungsmittel, das aus Verbindungen gemäß der allgemeinen Struktur ausgewählt ist, in einer Konzentration im Bereich von 0,01 Gewichts-% bis 20 Gewichts-% aufweist, bezogen auf die Gesamtzusammensetzung.

3. Zusammensetzung nach Anspruch 1 und 2, **dadurch gekennzeichnet, dass** sie Carnosin in einer Konzentration von 0,01 Gewichts-% bis 5 Gewichts-% aufweist, bezogen auf die Gesamtzusammensetzung.

4. Zusammensetzung nach einem der vorhergehenden Ansprüche, **dadurch gekennzeichnet, dass** sie weiteres Haarkonditionierungsmittel, ausgewählt aus kationischen Tensiden gemäß der allgemeinen Struktur 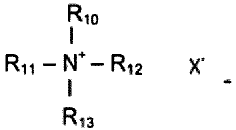 wobei R₁₀ für eine gesättigte oder ungesättigte, verzweigte oder unverzweigte Alkylkette mit 8 bis 24 C-Atomen, vorzugsweise 10 bis 24 C-Atomen und insbesondere 12 bis 22 C-Atomen steht und R₁₁ für eine ungesättigte oder gesättigte, verzweigte oder unverzweigte Alkylkette mit 1 bis 24 C-Atomen steht und R₁₂ und R₁₃ für eine niedere Alkylkette mit 1 bis 4 Kohlenstoffatomen stehen, welche mit einer oder mehreren Hydroxygruppen substituiert sein können, und X für ein Anion wie Chlorid, Bromid, Methosulfat steht, kationische Polymere, Öl und/oder ölige Substanzen und Polyole aufweist.

5. Zusammensetzung nach Anspruch 4, **dadurch gekennzeichnet, dass** das Öl und/oder die öligen Substanzen aus Silikonölen, Fettalkoholethern (Dialkylether) und Fettsäurefettalkoholestern ausgewählt ist/sind.

6. Zusammensetzung nach einem der vorhergehenden Ansprüche, **dadurch gekennzeichnet, dass** sie mindestens einen Direktfarbstoff aufweist.

7. Zusammensetzung nach einem der vorhergehenden Ansprüche, **dadurch gekennzeichnet, dass** sie mindestens einen Fettalkohol der folgenden Formel
R₂₀-OH
wobei R₂₀ für eine gesättigte oder ungesättigte, verzweigte oder unverzweigte Fettacylkette mit 8 bis 24 C-Atomen steht, und gegebenenfalls mindestens ein nichtionisches Tensid als Emulgator aufweist.

8. Zusammensetzung nach einem der vorhergehenden Ansprüche, **dadurch gekennzeichnet, dass** sie eine oder mehrere Verbindungen aufweist, ausgewählt aus Proteinhydrolysat, Polyphenol, Ubichinon der allgemeinen Struktur 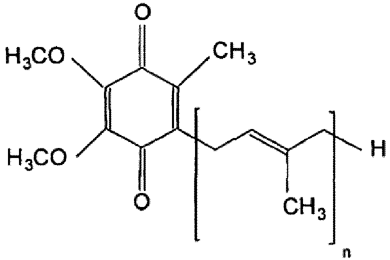 wobei n eine Zahl von 1 bis 10 ist, UV-Filter, organischem Lösungsmittel, Verdickungsmittel und Chelatbildner.

9. Zusammensetzung nach einem der vorhergehenden Ansprüche, **dadurch gekennzeichnet, dass** sie einen pH-Wert im Bereich von 2,0 bis 8,0 aufweist.

10. Verwendung der Zusammensetzung nach Anspruch 1 bis 9 zum Konditionieren von Haar und insbesondere zum Bewahren oder Verbessern des Glanzes, des Volumens, der Struktur, der Elastizität und der Bearbeitbarkeit von Haar.

11. Kit für Haar, **dadurch gekennzeichnet, dass** es zwei oder mehr Produkte aufweist, wobei mindestens ein Produkt eine Zusammensetzung nach Anspruch 1 bis 9 aufweist.

## Revendications

1. Composition de conditionnement pour cheveux **caractérisée en ce qu'**elle comprend au moins un agent de conditionnement capillaire choisi à partir des composés ayant la structure générale
R₁-A-R₂-B
dans laquelle R₁ est un groupe alkyle saturé ou non saturé, linéaire ou ramifié, de 8 à 24 atomes de carbone, R₂ est un groupe alkyle linéaire ou ramifié de 1 à 4 atomes de carbone, A est un groupe choisi à partir d'un atome d'O, d'un groupe 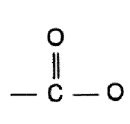 et d'un groupe 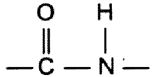 de préférence un atome d'O, et B est choisi à partir d'un groupe 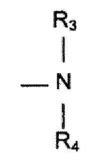 où R₃ et R₄ sont identiques ou différents, sont un atome H ou un groupe alkyle de 1 à 4 atomes de C, un groupe hydroxyle alkyle de 1 à 4 atomes de C et un groupe di hydroxyle alkyle de 2 à 4 atomes de C,
d'un groupe 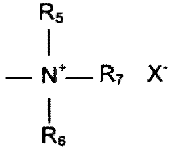 R₅ et R₆ sont identiques ou différents, un groupe alkyle de 1 à 4 atomes de C, un groupe hydroxyle alkyle de 1 à 4 atomes de C et un groupe di hydroxyle alkyle de 2 à 4 atomes de C, R₇ est un groupe alkyle de 1 à 4 atomes de C, un groupe hydroxyle alkyle de 1 à 4 atomes de C ou un groupe di hydroxyle alkyle de 2 à 4 atomes de C et
-R₂-A-R₁
où R₁, A et R₂ ont la signification donnée ci-dessus et X est un chlorure, un bromure, un méthosulfate, et une carnosine.

2. Composition selon la revendication 1, **caractérisée en ce qu'**elle comprend au moins un agent de conditionnement capillaire choisi à partir des composés selon la structure générale à une concentration dans une gamme de 0,01 à 20 % en poids, calculée par rapport à la composition totale.

3. Composition selon les revendications 1 et 2, **caractérisée en ce qu'**elle comprend de la carnosine à une concentration de 0,01 à 5 % en poids calculée par rapport à la composition totale.

4. Composition selon l'une quelconque des revendications précédentes, **caractérisée en ce qu'**elle comprend un agent de conditionnement capillaire complémentaire choisi à partir des tensioactifs cationiques selon la structure générale 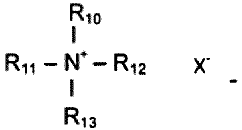 où R₁₀ est une chaîne alkyle saturée ou non saturée, linéaire ou ramifiée, de 8 à 24 atomes de C, préférablement de 10 à 24 atomes de carbone et plus préférablement de 12 à 22 atomes de carbone, et R₁₁ est une chaîne alkyle saturée ou non saturée, linéaire ou ramifiée, de 1 à 24 atomes de C et R₁₂ et R₁₃ sont des chaînes alkyles plus courtes de 1 à 4 atomes de carbone qui peuvent être substituées par un ou par plusieurs groupes hydroxyles, et X est un anion tel qu'un chlorure, un bromure, un méthosulfate, un polymère cationique, de l'huile et/ou des substances huileuses et des polyols.

5. Composition selon la revendication 4, **caractérisée en ce que** l'huile et/ou les substances huileuses (sont) choisies à partir des huiles silicones, des éthers d'alcool gras (dialkyle éther), les esters d'alcool gras et d'acide gras.

6. Composition selon l'une quelconque des revendications précédentes, **caractérisée en ce qu'**elle comprend au moins un colorant direct.

7. Composition selon l'une quelconque des revendications précédentes, **caractérisée en ce qu'**elle comprend au moins un alcool gras selon la formule suivante
R₂₀-OH
où R₂₀ est une chaîne d'acyle gras saturée ou non saturée, linéaire ou ramifiée, de 8 à 24 atomes de C et éventuellement au moins un tensioactif non ionique en tant qu'émulsifiant.

8. Composition selon l'une quelconque des revendications précédentes, **caractérisée en ce qu'**elle comprend un ou plusieurs des composés choisi(s) à partir d'un hydrolysat de protéine, de polyphénol, d'une ubiquinone de structure générale 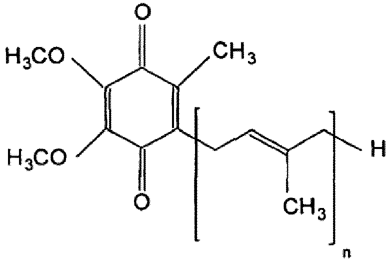 où n est un nombre entre 1 et 10, d'un filtre UV, d'un solvant organique, d'un agent épaississant et d'un agent de chélation.

9. Composition selon l'une quelconque des revendications précédentes, **caractérisée en ce que** son pH se situe dans la gamme de 2,0 à 8,0.

10. Utilisation de la composition selon les revendications 1 à 9, pour conditionner les cheveux et particulièrement pour conserver ou améliorer l'éclat, le volume, la structure, l'élasticité et la docilité des cheveux.

11. Kit pour cheveux **caractérisé en ce qu'**il comprend deux produits ou davantage où au moins un produit comprend une composition selon les revendications 1 à 9.
